# NEUE EUROPÄISCHE PATENTSCHRIFT

(11) **EP 2 010 252 B2**
(45) Veröffentlichungstag und Bekanntmachung des Hinweises auf die Entscheidung über den Einspruch: **18.08.2021**
(45) Hinweis auf die Patenterteilung: 22.12.2010
(21) Anmeldenummer: 07720074.9
(22) Anmeldetag: 12.04.2007
(51) Int. Cl.: A61M 5/20, A61M 5/24, A61M 5/315

(54) **Injektionsvorrichtung mit Spannfeder und Spannelement**
Injection device with tensioning spring and tensioning element
Dispositif d'injection avec ressort de serrage et element de serrage

(30) Priorität: 12.04.2006 DE 102006017209
(43) Veröffentlichungstag der Anmeldung: 07.01.2009
(73) Patentinhaber: TecPharma Licensing AG, 3401 Burgdorf (CH)
(72) Erfinder: MOSER, Ulrich, CH-3412 Heimiswil (CH)
(74) Vertreter: SSM Sandmair
(86) Internationale Anmeldenummer: PCT/CH2007/000177
(87) Internationale Veröffentlichungsnummer: WO 2007/115424

(56) Entgegenhaltungen:
- EP-A- 1 759 728
- EP-A1- 0 824 923
- EP-A1- 0 943 349
- WO-A1-99/02037
- WO-A2-92/19296
- FR-A1- 2 728 172
- US-A- 2 392 196
- US-A- 5 092 842
- US-A1- 2005 049 550

## Beschreibung

Die vorliegende Erfindung bezieht sich auf eine Vorrichtung zur Abgabe einer Substanz, insbesondere eine Injektionsvorrichtung oder einen Pen, der für die dosierte Verabreichung eines injizierbaren Produktes zum Beispiel aus einer Zweikammer-Ampulle eine Feder aufweist. Das Injektionsgerät dient vorzugsweise der Selbstverabreichung des Produkts, insbesondere als Einweg-Injektor oder Autopen.

Aus der US 5,104,380 ist ein Injektionsgerät bekannt, bei dem das Einstechen der am Gerät befestigten Injektionsnadel manuell und die nachfolgende Ausschüttung eines Wirkstoffes aus dem Gerät automatisch durch einen Antrieb erfolgt. Der Antrieb wird von einer Feder gebildet, die durch eine Bewegung eines Dosiergliedes zum Einstellen einer gewünschten Verabreichungsdosis gespannt wird. Die Feder wird in dem gespannten Zustand bis zur Aktivierung arretiert. Durch die Aktivierung treibt sie automatisch einen Ausschüttmechanismus an, der den Wirkstoff durch die Injektionsnadel austreibt.

Es ist eine Aufgabe der vorliegenden Erfindung, eine Injektionsvorrichtung vorzuschlagen, welche die Handhabung der Injektionsvorrichtung vereinfachen.

Aus der FR 2728172 A1 ist ein Autoinjektionsgerät bekannt, bei welchem eine Feder durch einen Stössel vorgespannt in das Gerät gebracht wird.

Aus der US 2005/049550 A1 ist ein Verfahren zum Vorbereiten einer Injektionsvorrichtung bekannt, wobei eine Feder gespannt wird. Die Feder ist von einer Federhülle umhüllt, welche als Federwiderlager dient und Verriegelungselemente aufweist, um die Federhülle an das Abmischgerät zu koppeln. Die Feder ist während dem Spannen immer an dem Abmischgerätgehäuse und an der Federhülle abgestützt. Aus WO 92/19296 ist eine Injektionsvorrichtung bekannt, bei der eine distal angeordnete Aussenhuelse ueber einen Ampullenhalter geschoben werden muss, um ein Sperrelement zur Freigabe der Ausschuettfeder zu loesen. Aus US 5,092,842 ist eine Injektionsvorrichtung bekannt, bei der ein aussen am Gehaeuse angeordneter Arm zur Ausloesung einer Injektion radial einwaerts gedrueckt werden muss, um ein Vorschieben der Ausschuettfeder zu ermoeglichen. EP 0 824 923 A1 offenbart einen Autoinjektor mit einem seitlich angeordneten Ausloeseknopf, der indirekt auf die Kolbenstange des Autoinjektors wirkt.

Diese Aufgabe wird durch eine Injektionsvorrichtung gemäss Patentanspruch 1 gelöst. Vorteilhafte Ausführungsformen ergeben sich aus den abhängigen Patentansprüchen.

Eine erfindungsgemässe Injektionsvorrichtung weist eine Aufnahmevorrichtung für eine eine abzugebende Substanz enthaltende Ampulle auf, in welcher die abzugebende Substanz gespeichert werden kann oder in einem Behältnis, zum Beispiel in einer einfachen oder Zweikammer-Ampulle, bevorratet werden kann, so dass die Aufnahmevorrichtung als Ampullenhalter ausgebildet ist.

Die Aufnahmevorrichtung ist relativ zur Injektionsvorrichtung oder einem Gehäuse des Pens bewegbar, wobei ein die Aufnahme oder den Ampullenhalter bildendes Element der Injektionsvorrichtung in diese oder in einen Teil davon, zum Beispiel in das Gehäuse der Injektionsvorrichtung, einschiebbar oder einschraubbar ist. Bei Verwendung einer bekannten Zweikammer-Ampulle können durch den Einschub- oder Einschraubvorgang die in der Zweikammer-Ampulle enthaltenen Substanzen abgemischt und somit für die Abgabe an einen Patienten vorbereitet werden. In oder an der Injektionsvorrichtung ist eine Feder als ein Spannelement vorgesehen, welche sich an einer Seite gegen ein Element in oder an der Injektionsvorrichtung oder an deren Gehäuse, wie zum Beispiel einem hierzu vorgesehenen Ring oder eine Scheibe, abstützt und welche erfindungsgemäss an der von der abstützenden Seite oder Fläche wegweisenden Richtung mittels eines Spann- oder Koppelelementes, welches mit dem Ampullenhalter verbunden oder gekoppelt werden kann, durch das Einschieben oder Eindrehen des Ampullenhalters gespannt werden kann. Die gespannte Feder kann in dem gespannten Zustand durch ein Koppelelement, welches als Schubhülse oder strokesleeve ausgebildet sein kann, gehalten werden, bis das Koppelelement, welches die Federspannung durch eine Kopplung mit dem Ampullengehäuse hält, durch Betätigung eines Auslöseknopfes von dem Ampullengehäuse entkoppelt wird und ein Entspannen der Feder ermöglicht. Die Feder wirkt über das Koppelelement auf die Kolbenstange ein und drueckt diese in distale Richtung, so dass durch eine Bewegung der Kolbenstange in distale Richtung ein oder mehrere Stopfen in die Ampulle eingeschoben werden, um eine in der Ampulle enthaltene und zum Beispiel beim Einbringen der Ampulle abgemischte Substanz aus der Ampulle zu verdrängen und somit für die Injektion auszuschütten.

Das Koppelelement kann mit dem Ampullenhalter durch eine lösbare Verbindung durch ein Halte- oder Rastelement verbunden sein oder erst beim Einsetzen des Ampullenhalters mit diesem koppeln. Das Koppelelement weist mindestens ein und bevorzugt zwei oder mehr Halte- oder Rastelemente auf, welche ein Koppeln oder Verrasten mit dem Ampullenhalter und/oder der Kolbenstange ermöglichen. Hierzu können die Halte- oder Rastelemente radial nach innen und/oder nach aussen vorgespannt sein und so beispielsweise bei einem zylinderförmigen oder hülsenförmigen Koppelelement nach innen, zum Beispiel in Richtung auf die Kolbenstange, und/oder nach aussen, zum Beispiel in Richtung auf ein Gehäuse der Injektionsvorrichtung, drücken.

Ein oder mehrere Halte- oder Rastelemente des Koppelelementes können abgeschrägte, also zum Beispiel auf der Längsachse der Injektionsvorrichtung nicht senkrecht stehende, oder konisch zulaufende Flächen oder Teilstücke aufweisen. Ein zum Beispiel in axialer Richtung der Injektionsvorrichtung auf das Koppelelement drückendes Glied, wie zum Beispiel ein mit dem Auslöseknopf oder Einstellring verbundenes Element, löst ein das Koppelelement in einer axial fixierten Stellung haltendes Halte- oder Rastelement aus einer Kopplungs- oder Rastverbindung zum Beispiel mit dem Gehäuse der Injektionsvorrichtung, um das Koppelelement freizugeben. Das Koppelelement wird zum. Beispiel durch den Druck der durch das Einschieben des Ampullenhalters vorgespannten Feder in distale Richtung der Injektionsvorrichtung bewegt. Dabei kann diese Vorschubbewegung des Koppelelementes zum Beispiel mittels einer Kopplung oder Rastverbindung auf die Kolbenstange übertragen werden.

Bevorzugt ist das Koppelelement verdrehsicher auf der Kolbenstange gelagert. Beispielsweise kann die Kolbenstange ein nicht rundes Profil aufweisen und zum Beispiel im Querschnitt rechteckig oder quadratisch sein, wobei das Koppelelement zum Beispiel hülsenförmig ausgebildet sein kann und einen Führungsbereich aufweisen kann, in welchem die Kolbenstange axial verschiebbar geführt werden kann, so dass das Koppelelement in axialer Richtung entlang der Kolbenstange bewegbar ist. Ebenso kann in oder auf der Kolbenstange in Längsrichtung eine Nut oder ein Steg vorgesehen sein, wobei am Koppelelement ein entsprechendes Gegenelement, also zum Beispiel ein Steg oder eine Nut, vorgesehen sein kann.

Vorzugsweise ist der Ampullenhalter mittels eines Gewindes mit der Injektionsvorrichtung oder einem Gehäuse davon verbunden, so dass der Ampullenhalter in die Injektionsvorrichtung eingeschraubt werden kann. Bevorzugt weist der Ampullenhalter ein Aussengewinde auf, welches in ein Innengewinde der Injektionsvorrichtung eingedreht werden kann. Ebenso ist auch ein einfaches Einschieben des Ampullenhalters in die Injektionsvorrichtung ohne relative Drehbewegung möglich.

Der zur Einstellung der abzugebenden Dosis an der Injektionsvorrichtung vorgesehene Dosiseinstellring ist vorzugsweise blockiert, d.h. relativ zur Injektionsvorrichtung nicht drehbar oder nur sehr eingeschränkt drehbar, bis die Ampulle vollständig eingesetzt ist. Hierfür sind an dem Dosiseinstellring ein oder mehrere Anschläge vorgesehen, die im Zustand eingesetzten Ampullenhalters ungehindert beweglich sind. Vorzugsweise laufen die Anschläge zumindest teilweise innerhalb einer Nut, insbesondere einer Ringnut. Die Nut kann an einer im Inneren des Gehäuses angeordneten Funktionshülse vorgesehen sein. Im Zustand ohne eingesetzten Ampullenhalter kommen die Anschläge gegenüber einem, bzw. mehreren Gegenanschlägen zu liegen, so dass ihre Bewegung blockiert wird. Die Gegenanschläge können durch das Einsetzen des Ampullenhalters aus der Bewegungsbahn der Anschläge des Dosiseinstellrings gebracht werden, so dass der Dosiseinstellring zur Dosiseinstellung frei gedreht werden kann. Hierfür kann z.B. die Funktionshülse als Ganzes gegenüber dem Dosiseinstellring verschoben werden oder die Funktionshülse kann geteilt sein, so dass sich nur ein Teil der Funktionshülse beim Einsetzen des Ampullenhalters verschiebt, beispielsweise derart, dass Gegenanschläge aus der Ringnut herausbewegt werden.

Vorzugsweise ist der Auslöseknopf für den Ausschüttvorgang so mit dem Dosiseinstellring gekoppelt, dass beim Einstellen einer Dosis mittels des Einstellringes der Auslöseknopf aus dem Gehäuse der Injektionsvorrichtung herausgefahren wird. Hierzu kann beispielsweise eine gewindeartige Kopplung zwischen Auslöseknopf und Einstellring vorgesehen sein, so dass beispielsweise ein verdrehsicher gelagerter Auslöseknopf durch eine Drehung des Einstellringes aus der Injektionsvorrichtung herausgedreht wird.

In einer Ausführungsform ist der Auslöseknopf beispielsweise verdrehgesichert, aber axial beweglich an dem Dosiseinstellring angeordnet. Durch die Drehbewegung des Dosiseinstellrings kann der Auslöseknopf entlang einer Führungskulisse axial gegenüber dem Dosiseinstellring bewegt werden. Als Führungskulisse eignen sich z.B. schräge Fläche, die aufeinander abgestimmt am Auslöseknopf und an einem zum Dosiseinstellring drehfesten Element, wie etwa der Funktionshülse, angeordnet sind. Vorzugsweise ist der Auslöseknopf soweit in dem Gehäuse, bzw. in dem Dosiseinstellring versenkt angeordnet, dass er unzugänglich ist, solange er nicht durch das Einsetzen des Ampullenhalters, bzw. das Drehen des Dosiseinstellrings, aktiviert wird.

Nach der Beendigung des Einstellvorganges ist der Auslöseknopf in einer aktivierbaren Stellung. Durch Betätigung des Knopfes kann eine Ausschüttung ausgelöst werden. Vorzugsweise wird der Auslöseknopf dabei wieder in eine versenkte Stellung gebracht, in der er für weitere Betätigungen unzugänglich ist. In einer besonders bevorzugten Ausführung ist auch der Dosiseinstellring nach der Ausschüttung des Wirkstoffes, d.h. der Betätigung des Auslöseknopfs wieder in einer blockierten Stellung. Die Injektionsvorrichtung ist dann nach einer einzigen Ausschüttung für weitere Betätigungen vollständig blockiert. Es kann weder eine weitere Dosiseinstellung noch eine erneute Betätigung des Auslöseknopfes erfolgen. Der Pen ist vollständig gesichert.

Zum Vorbereiten einer Injektionsvorrichtung mit einer Aufnahmevorrichtung für eine abzugebende Substanz wird eine Feder, die sich an einem Teil der Injektionsvorrichtung abstützt, durch eine relative Bewegung der Aufnahmevorrichtung zur Injektionsvorrichtung oder zu einem Teil der Injektionsvorrichtung über ein Koppelelement gespannt, wobei das Koppelelement zur Koppelung der Aufnahmevorrichtung mit der Feder dient. Die Injektion erfolgt dadurch, dass die gespannte Feder nach Betätigung eines Auslöseknopfes wieder entspannt wird und eine Kraft auf eine Kolbenstange ausübt, welche in die Ampulle eingeschoben wird.

Die Erfindung wird nachfolgend anhand eines Ausführungsbeispieles beschrieben. Es zeigen:
- Figuren 1 A und 1B: senkrecht aufeinanderstehende Längsschnittansichten einer Injektionsvorrichtung mit entspannter Ausschüttfeder vor dem Eindrehen der Ampulle;
- Figuren 1C und 1D: aufeinander senkrecht stehende Seitenansichten der Injektionsvorrichtung gemäss Figuren 1A und 1B;
- Figuren 2A und 2B: Längsschnittansichten einer Injektionsvorrichtung nach dem Eindrehen der Ampulle mit gespannter Ausschüttfeder;
- Figuren 2C und 2D: aufeinander senkrecht stehende Seitenansichten der Injektionsvorrichtung gemäss Figuren 2A und 2B;
- Figuren 3A und 3B: Längsschnittansichten einer Injektionsvorrichtung nach dem Dosieren mittels des Einstellringes mit ausgefahrenem Auslöseknopf; und
- Figuren 3C und 3D: aufeinander senkrecht stehende Seitenansichten der Injektionsvorrichtung gemäss Figuren 3A und 3B;
- Figuren 4A und 4B: Längsschnittansichten einer Injektionsvorrichtung in Endstellung nach dem Eindrücken des Auslöseknopfes und Auslösen der Feder zum Ausschütten der Dosis;
- Figuren 4C und 4D: aufeinander senkrecht stehende Seitenansichten der Injektionsvorrichtung gemäss Figuren 4A und 4B.

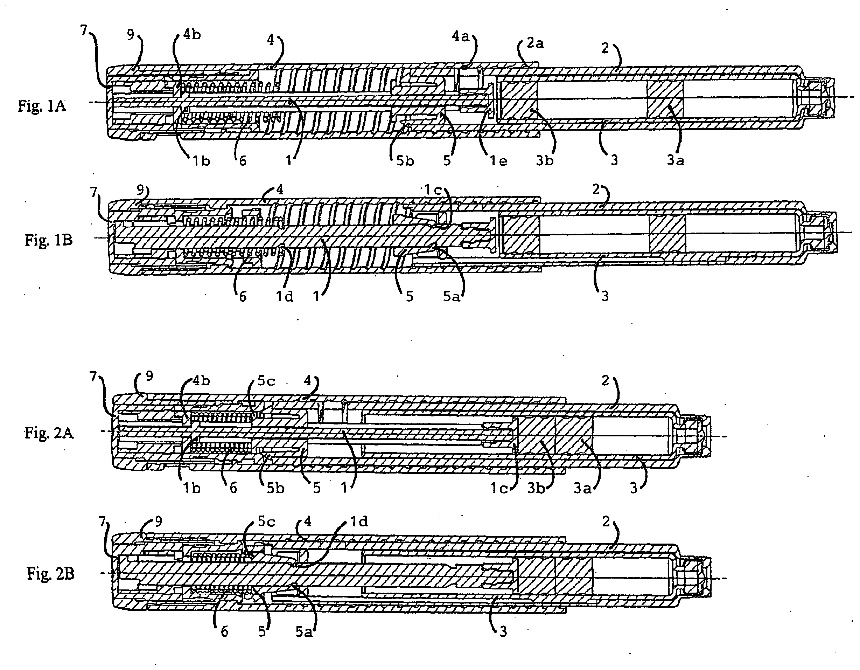

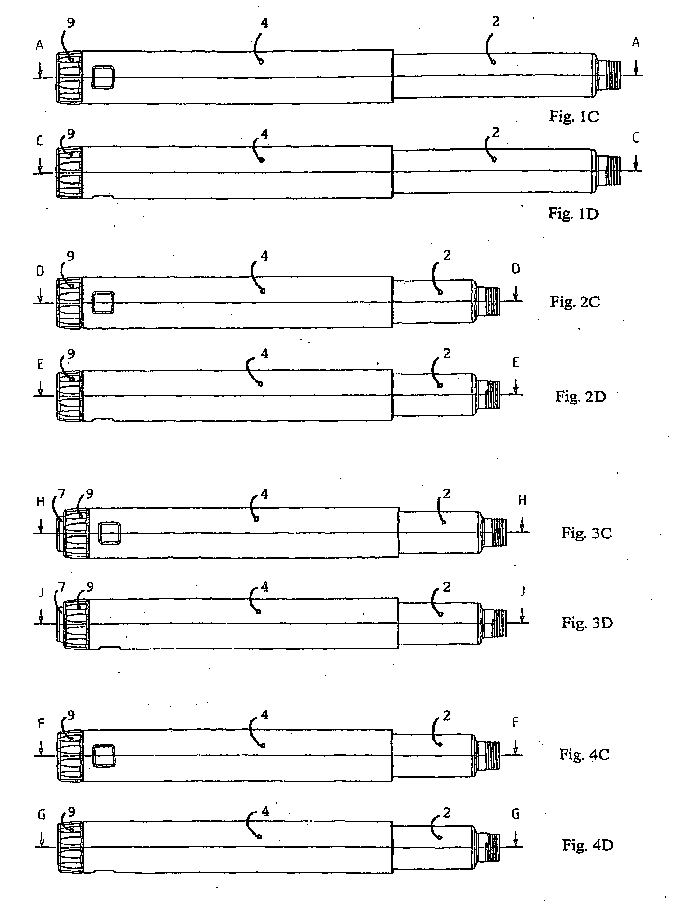

Figur 1A zeigt eine Querschnittsansicht in Längsrichtung einer Ausführungsform einer erfindungsgemässen in Figur 1C gezeigten Injektionsvorrichtung, wobei ein Ampullenhalter 2, in welchen eine Ampulle 3 eingelegt ist, zum Teil mittels des Aussengewindes 2a des Ampullenhalters 2 in ein Innengewinde 4a in das Gehäuse 4 der Injektionsvorrichtung bzw. des Pens eingeschraubt ist. Die Ampulle 3 ist eine Zweikammer-Ampulle und weist zwei Stopfen 3a und 3b auf, welche zwei abzumischende und in Ausgangsstellung voneinander getrennte Substanzen in der Ampulle 3 abschliessen. Eine Kolbenstange 1 ist in axialer Richtung gegen eine Verschiebung in proximale Richtung durch einen von der Kolbenstange 1 radial abstehenden Ring 1b gesichert, welcher an eine durch Stege 4b gebildeten Durchtrittsöffnung, die kleiner als der Aussendurchmesser des Ringes 1 b ist, anliegt. Auf der Kolbenstange 1 ist als Koppelelement eine Schubhülse oder strokesleeve 5 verdrehsicher und auf der Kolbenstange 1 verschiebbar angeordnet.

Figur 1B zeigt den in Figur 1A gezeigten Pen in Querschnittsansicht in einer in Figur 1D gezeigten Schnittebene, welche senkrecht auf der in Figur 1A gezeigten Schnittebene steht. Die Schubhülse 5 weist radial nach innen vorgespannte Rastelemente 5a auf, welche in Ausnehmungen 1c der Kolbenstange 1 eingreifen und somit in der Grundstellung entspannt sind. Eine Feder 6 stützt sich gegen eine Anlagefläche 4b im Inneren der Injektionsvorrichtung ab und ist in der Figur 1 gezeigten Grundstellung entspannt.

Beim Eindrehen des Ampullenhalters 2 in das Gehäuse 4 kommt der hintere Stopfen 3b der Ampulle 3 in Anlage an ein Anlageelement 1e der Kolbenstange 1 und wird bei einem weiteren Eindrehen des Ampullenhalters 2 relativ zur Ampulle 3 in diese eingeschoben, was zu einer Abmischung der in der Zweikammer-Ampulle 3 enthaltenen Substanzen führt.

Figur 2A zeigt die in Figur 1A gezeigte Injektionsvorrichtung nach dem Eindrehen des Ampullenhalters 2 in das Gehäuse 4. Die Schubhülse 5, welche mittels radial nach aussen vorgespannten Halteelementen 5b mit dem Ampullenhalter 2 gekoppelt ist, wurde durch das Eindrehen des Ampullenhalters 2 in proximale Richtung der Injektionsvorrichtung verschoben. Sie führt somit zu einer Spannung der Feder 6 zwischen den Anlageflächen 4b und 5c, welche bevorzugt während der gesamten und zumindest während eines Teils der Ampulleneinschubbewegung in Anlage mit der proximalen Fläche 5c der Schubhülse 5 ist und durch die Schubhülse 5 zusammengedrückt wurde und somit vorgespannt ist. Die radial nach innen vorgespannten Eingriffselemente 5a der Schubhülse 5 wurden aus den Ausnehmungen 1c der Kolbenstange 1 herausgezogen und greifen in der in Figur 2B gezeigten Position in die hinteren Ausnehmungen 1 d der Kolbenstange 1 ein.

Der Dosiseinstellring ist vor dem Eindrehen des Ampullenhalters 2 nicht drehbar gegenüber dem Gehäuse 4. Hierfür weist der Dosiseinstellring Anschläge auf, die an in Umfangsrichtung angeordneten flexiblen Armen vorgesehen sind. Die Anschläge kommen gegenüber einer Ringnut zu liegen, die an einer Funktionshülse vorgesehen ist. Bei einer Dosierbewegung werden die Anschläge durch eine Bewegung der flexiblen Arme in die Ringnut gedrückt, da den Armen gegenüberliegend eine Verzahnung vorgesehen ist, entlang der die Arme bewegt werden, wobei ein hörbares Klickgeräusch erzeugt wird. Laufen die flexiblen Arme über die Zähne, werden sie bei jedem Zahn in die Ringnut gedrückt. Die Funktionshülse ist gegenüber dem Gehäuse verdrehgesichert und zweiteilig ausgebildet, wobei die beiden Teile gegeneinander teleskopartig verschieblich sind. In axial auseinander gezogenem Zustand der Teile der Funktionshülse kommen Gegenanschläge innerhalb der Ringnut zu liegen, die den Anschlägen an dem Dosiseinstellring gegenüberliegen und ein Drehen des Dosiseinstellrings blockieren. In axial zusammen geschobenem Zustand der Teile der Funktionshülse kommen die Gegenanschläge ausserhalb der Bahn der Ringnut zu liegen, so dass der Dosiseinstellring drehbar ist. Die Verschiebung der Teile der Funktionshülse zueinander erfolgt durch das Eindrehen des Ampullenhalters 2. Dadurch ist der Dosiseinstellring erst drehbar, wenn der Ampullenhalter 2 eingesetzt ist.

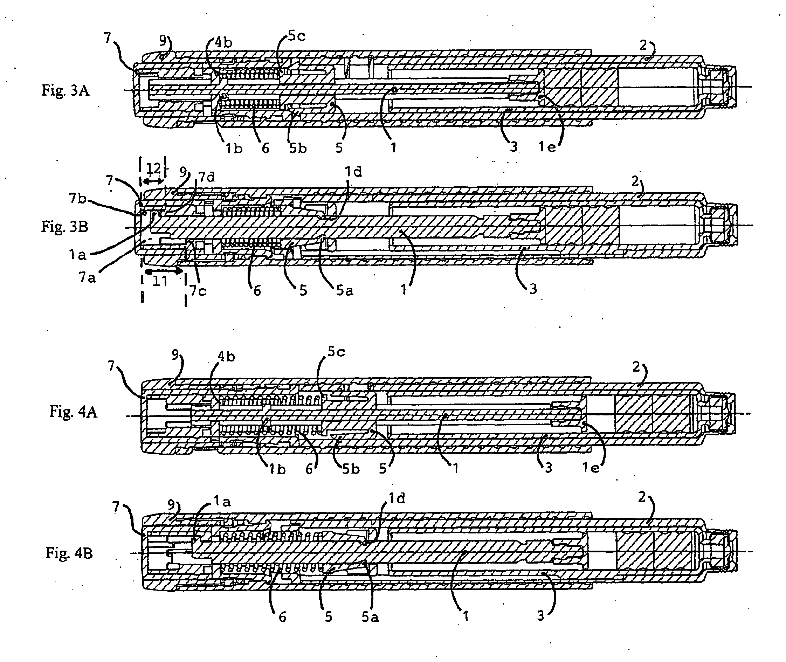

Figur 3A zeigt die in Figur 2A gezeigte Injektionsvorrichtung, nachdem der Einstellring 9 zum Einstellen der Dosis gedreht wurde, wobei der Auslöseknopf 7 durch die Einstellbewegung des Einstellringes 9 aus der Injektionsvorrichtung mittels einer Kulissenführung ausgefahren wird. Hierfür ist an einer proximalen Stirnseite der Funktionshülse proximal vorstehender Vorsprung mit einer schrägen Fläche vorgesehen. An einer distalen Stirnseite des Auslöseknopfes ist ein distal vorstehender Vorsprung mit einer schrägen Fläche vorgesehen, die zu der Fläche der Funktionshülse komplementär ist. Bei einem Verdrehen des Dosiseinstellrings wird der Auslöseknopf mitgenommen und gegenüber der Funktionshülse gedreht, wobei die schrägen Fläche aufeinander abrutschen und somit der Auslöseknopf gegenüber der Funktionshülse und dem Dosiseinstellring axial verschoben wird. Der Auslöseknopf wird dadurch aus einem versenkten Zustand in einen betätigbaren Zustand gebracht.

Beim Eindrücken des Auslöseknopfes in die Injektionsvorrichtung wirkt die Funktionshülse auf das Schubelement 5 und löst die Feder aus. Die Funktionshülse bildet in diesem Fall ein Freigabeelement 8.

An der Innenseite des Auslöseknopfes 7 sind, wie in Figur 3B ersichtlich, Nuten unterschiedlicher axialer Länge 11 und 12 vorgesehen, wie beispielhaft durch die Nuten 7a und 7b gezeigt, welche die maximal mögliche Axialbewegung der Kolbenstange 1 festlegen, die an ihrem proximalen Ende eine Nase 1 a aufweist, die nach Freigabe der durch die Feder 6 vorgespannten Kolbenstange 1 an einen die jeweiligen Nuten 7a, 7b begrenzenden Anschlag 7c oder 7d kommt und somit die maximal mögliche Aussch üttdosis festlegt.

Wird der Auslöseknopf 7 in die Injektionsvorrichtung eingedrückt, wie in Figur 4A gezeigt, so wird das radial nach aussen vorgespannte Halteelement 5b, welches die Schubhülse 5 an dem proximalen Ende des Ampullenhalters 2 hält, mittels dem mit dem Knopf 7 zusammenwirkenden Freigabeelements 8, das auf eine abgeschrägte Fläche 5c des Halteelements 5b drückt und dieses somit radial nach innen schiebt, ausser Eingriff mit dem Ampullenhalter 2 gebracht, so dass die Schubhülse 5 in axialer Richtung nicht mehr relativ zur Injektionsvorrichtung arretiert ist. Die auf die Schubhülse 5 drückende Feder 6 schiebt die Schubhülse 5 in distaler Richtung. Da die Schubhülse 5 mittels der radial nach innen vorgespannten Rastelemente 5a, welche in die Ausnehmungen 1d der Kolbenstange 1 eingreifen, die Kolbenstange 1 mitnimmt, wird die Kolbenstange 1 in die Ampulle 3 eingeschoben und drückt so auf den hinteren Stopfen 3b, was zu einer Ausschüttung der in der Ampulle 3 enthaltenen abgemischten Substanz führt.

In der in Figur 4B gezeigten Position nach dem Ausschütten der Dosis aus der Ampulle 3 wird der Auslöseknopf 7 von der Nase 1a der Kolbenstange 1 in der Injektionsvorrichtung gehalten und kann nicht mehr herausgezogen werden, so dass die Injektionsvorrichtung nicht mehr für eine weitere Injektion verwendet werden kann.

## Patentansprüche

1. Injektionsvorrichtung mit einer Aufnahmevorrichtung für eine abzugebende Substanz, wobei die Aufnahmevorrichtung als Ampullenhalter (2) ausgebildet ist, welche relativ zur Injektionsvorrichtung oder einem Teil davon bewegbar ist, einer Ausschüttfeder (6), die sich an einem Teil der Injektionsvorrichtung abstützen kann, einem Koppelelement (5) zur Kopplung der Aufnahmevorrichtung (2) mit der Ausschüttfeder (6), und einem Auslöseknopf (7) zum Auslösen einer Injektion, wobei die Feder (6) durch eine Einführbewegung der Aufnahmevorrichtung (2) in die Injektionsvorrichtung spannbar ist, wobei die Feder zum Ausschütten einer Substanz über das Koppelelement (5) auf eine Kolbenstange (1) der Injektionsvorrichtung wirkend ausgebildet ist, wobei das Koppelelement (5) mindestens ein Halte- oder Rastelement (5a, 5b) aufweist, um das Koppelelement (5) lösbar mit der Aufnahmevorrichtung (2) und/oder einer Kolbenstange (1) der Injektionsvorrichtung zu verbinden, wobei das Koppelelement (5) eingerichtet ist, die Ausschüttfeder (6) in einem gespannten Zustand zu halten und durch Betätigung des Auslöseknopfes (7) ein Entspannen der Ausschüttfeder (6) zu ermöglichen.

2. Injektionsvorrichtung nach Anspruch 1, wobei das Koppelelement (5) ein hülsenförmiges Element ist.

3. Injektionsvorrichtung nach dem vorhergehenden Anspruch, wobei das Koppelelement (5) ein konisch oder mit einer schräg verlaufenden Oberfläche ausgebildetes Kontaktelement (5c) aufweist.

4. Injektionsvorrichtung nach einem der vorhergehenden Ansprüche, wobei das Koppelelement (5) verdrehsicher auf einer Kolbenstange (1) gelagert ist.

5. Injektionsvorrichtung nach einem der vorhergehenden Ansprüche, wobei die Aufnahmevorrichtung (2) mit der Injektionsvorrichtung mittels eines Gewindes (2a, 4a) gekoppelt ist.

6. Injektionsvorrichtung nach einem der vorhergehenden Ansprüche, wobei ein Dosiseinstellring (9) blockiert werden kann, bis die Ampulle (3) vollständig eingesetzt ist.

7. Injektionsvorrichtung nach einem der vorhergehenden Ansprüche, wobei ein Auslöseknopf (7) der Injektionsvorrichtung durch ein Drehen eines Einstellringes (9) ausgefahren werden kann.

## Claims

1. An injection device with a device accommodating a substance to be delivered, wherein the accommodating device is designed as a vial holder (2), which is moveable relative to the injection device or a part thereof, a discharge spring (6), which can support itself on a part of the injection device, a coupling element (5) for coupling the accommodating device (2) to the discharge spring (6), and a trigger button (7) for triggering an injection, wherein the spring (6) can be tensioned when the accommodating device (2) is inserted into the injection device, wherein the spring for discharging a substance is designed to act on a piston rod (1) of the injection device via the coupling element (5), wherein the coupling element (5) has at least one retaining or locking element (5a, 5b), in order to detachably connect the coupling element (5) to the accommodating device (2) and/or a piston rod (1) of the injection device, wherein the coupling element (5) is set up to hold the discharge spring (6) in a tensioned state and to facilitate a relaxing of the discharge spring (6) by pressing the trigger button (7).

2. The injection device according to claim 1, wherein the coupling element (5) is a sleeve-shaped element.

3. The injection device according to the preceding claim, wherein the coupling element (5) has a contact element (5c) which is of a conical configuration or which has an inclined surface.

4. The injection device according to any of the preceding claims, wherein the coupling element (5) is mounted in a torsion-proof manner on a piston rod (1).

5. The injection device according to any of the preceding claims, wherein the accommodating device (2) is coupled to the injection device by means of a thread (2a, 4a).

6. The injection device according to any of the preceding claims, wherein a dose setting ring (9) can be blocked, until the vial (3) is completely inserted.

7. The injection device according to any of the preceding claims, wherein a trigger button (7) of the injection device can be extended by turning a setting ring (9).

## Revendications

1. Dispositif d'injection avec un dispositif de réception pour une substance à distribuer, dans lequel le dispositif de réception est réalisé sous la forme d'un support d'ampoule (2), lequel peut être déplacé par rapport au dispositif d'injection ou une partie de celui-ci, un ressort d'éjection (6), qui peut s'appuyer sur une partie du dispositif d'injection, un élément d'accouplement (5) pour accoupler le dispositif de réception (2) au ressort d'éjection (6), et un bouton de déclenchement (7) pour déclencher une injection, dans lequel le ressort (6) peut être tendu par un mouvement d'introduction du dispositif de réception (2) dans le dispositif d'injection, dans lequel le ressort est réalisé pour éjecter une substance par l'intermédiaire de l'élément d'accouplement (5) en agissant sur une tige de piston (1) du dispositif d'injection, dans lequel l'élément d'accouplement (5) présente au moins un élément de retenue ou d'encliquetage (5a, 5b), afin de relier l'élément d'accouplement (5) au dispositif de réception (2) et/ou à une tige de piston (1) du dispositif d'injection de manière libérable, dans lequel l'élément d'accouplement (5) est conçu pour retenir le ressort d'éjection (6) dans un état tendu et pour permettre une détente du ressort d'éjection (6) par actionnement du bouton de déclenchement (7).

2. Dispositif d'injection selon la revendication 1, dans lequel l'élément d'accouplement (5) est un élément en forme de manchon.

3. Dispositif d'injection selon la revendication précédente, dans lequel l'élément d'accouplement (5) présente un élément de contact (5c) réalisé de manière conique ou avec une surface s'étendant de manière inclinée.

4. Dispositif d'injection selon l'une quelconque des revendications précédentes, dans lequel l'élément d'accouplement (5) est monté sur une tige de piston (1) de manière bloquée en rotation.

5. Dispositif d'injection selon l'une quelconque des revendications précédentes, dans lequel le dispositif de réception (2) est accouplé au dispositif d'injection au moyen d'un filet (2a, 4a).

6. Dispositif d'injection selon l'une quelconque des revendications précédentes, dans lequel une bague de réglage de dose (9) peut être bloquée, jusqu'à ce que l'ampoule (3) soit entièrement insérée.

7. Dispositif d'injection selon l'une quelconque des revendications précédentes, dans lequel un bouton de déclenchement (7) du dispositif d'injection peut être sorti par une rotation d'une bague de réglage (9).
